# EUROPEAN PATENT SPECIFICATION

(11) **EP 0 546 762 B1**
(45) Date of publication and mention of the grant of the patent: **02.04.1997**
(21) Application number: 92311013.4
(22) Date of filing: 02.12.1992
(51) Int. Cl.: C12Q 1/68, C07H 21/04, C12P 19/34

(54) **Sexing method of bovine embryos**
Verfahren zur Geschlechtsbestimmung von Rinderembryos
Procédé pour la détermination du sexe d'embryons bovins

(30) Priority: 13.12.1991 JP 352032/91
(43) Date of publication of application: 16.06.1993
(73) Proprietor: ITOHAM FOODS INC., Kobe, Hyogo (JP)
(72) Inventor: Kudo, Toshiyuki, Kitasoma, Ibaraki (JP); Itagaki, Yoshiaki, Kitasoma, Ibaraki (JP); Sato, Seiji, Kitasoma, Ibaraki (JP); Suto, Shizuyo, Kitasoma, Ibaraki (JP); Nakamura, Toyoo, Kitasoma, Ibaraki (JP)
(74) Representative: Cresswell, Thomas Anthony

(56) References cited:
- WO-A-86/07095
- WO-A-89/01978
- WO-A-89/07154
- WO-A-91/00365
- THERIOGENOLOGY vol. 29, no. 1, January 1988, CALIFORNIA US page 242 ELLIS, S. ET AL. 'sex determination of bovine embroys using male specific dna probes'
- Cytogenet. Cell Genet. 56; 40-44 (1991)
- Genomics 6; 482-490 (1990)
- Biochemistry 16; 5329 (1977)
- Cell 37; 171-177 (1984)
- J.Reprod. Dev. 39; 55-63 (1993)

## Description

### Industrial Field of the Invention

The present invention relates to the method of sexing of bovine embryos and DNA primers for this method.

### Description of the Prior Art

The development of modern biotechnology has made artificial insemination a common technique in industrial animals. In particular, artificial insemination with frozen sperm has been established in cattle. Although sexing is an important technique from the industrial viewpoint, a simple and reliable experimental method has been rather difficult to develop in the field of cattle biotechnology.

WO89/01978 discloses synthetic oligonucleotides consisting of fragments of a molecular probe comprising a nucleotide sequence of 49 bp. The probe can be used for sexing the embryos or fetuses of ruminant mammals. WO86/07095 discloses further nucleic acid hybridization probes which have sequences complementary to sequences in segments of bovine-male specific DNA. Additional nucleic acid isolates capable of hybridizing only to Y-chromosome specific DNA sequences of ruminants, and methods for their use, are described in WO89/07154.

The following methods have so far been tried in order to sex bovine embryos experimentally.
1) Separation and Identification of Y-bearing Spermatozoa
   Since females are homozygotic and males are heterozygotic in mammals, including cattle, eggs are sexually homogeneous and spermatozoa consist of two populations. That is, ova fused with Y-bearing spermatozoa become male embryos and ova fused with X-bearing spermatozoa, female embryos. Therefore, if X- and Y-bearing spermatozoa can be separated, female and male embryos can be produced at will.
   (1) Separation by Sedimentation Method Utilizing Gravity Force or by Centrifugation in Density Gradients
      The X-chromosome comprises approximately 5% of the genome, and the Y-chromosome, about 3%. This slight difference implies that light spermatozoa are Y-bearing and heavy ones, X-bearing. However, definite separation is difficult, because the difference is small, the size of spermatozoa naturally fluctuates, and the specific gravity of spermatozoa depends to some extent on the degree of maturity.
   (2) Separation by Electrophoresis
      Generally, charges on the cytoplasmic membrane depend on the amount of sialic acid bound to glycoproteins. Separation by electrophoresis is based on the premise that there is a difference in surface charges between X- and Y-bearing spermatozoa. During meiosis, however, four spermatids covered by the Sertoli cell are open to each other through cytoplasmic bridges and they are therefore a kind of syncytium. This means that difference in surface charges are unlikely to occur; separation by this method is unlikely to be successful.
   (3) Discrimination by the Presence of F-body
      It is said that when spermatozoa are stained with quinacrine, a fluorescent dye, the Y-chromosome is specifically stained as the F-body in such species as the gorilla and human being and thus X- and Y-bearing spermatozoa can be discriminated. However, this may not hold true for other species, the ratio of spermatozoa having the F-body and those without it is not 50:50, and the Y-chromosome-specific staining itself is theoretically unlikely. This method seems doubtful, in principle.
   (4) Separation by Flow Cytometry
      Since the X-chromosome is slightly bigger than the Y-chromosome, after fluorescent staining, more intense light is emitted from the former. Utilizing this fact, X-chromosome-bearing spermatozoa can be separated from those having the Y-chromosome. Insemination of eggs with spermatozoa thus separated enables the production of male or female embryos at will.
      This flow-cytometric method has drawbacks. Spermatozoa need the laborious pretreatment of dye-staining and are irradiated with a laser beam; they might be damaged. Micro-insemination is necessary. The instrument is quite expensive. This method is not simple and accordingly has not been used commercially yet.
2) Anti-HY Antigen Antibody (HY Antibody) Method
   A skin graft from a male to a female in an inbred strain is rejected and drops off. The inverse graft from a female to a male is accepted. This is considered to be caused by a histocompatibility antigen called the HY antigen mapped on the Y-chromosome. The graft rejection reaction is under the control of the cellular immunity. However, anti-HY antibody was also found in the blood of a female rejecting a male graft, indicating the involvement of the humoral immunity.
   The HY antigen has been said to be expressed at the 8 cell stage in mice. There are reports that sexing of murine embryos was possible using the HY antibody derived from mice. It was also claimed that morphological changes of bovine embryos treated with the HY antibody from rats enabled discrimination of male embryos from female ones.
   Since the immunogenicity of the HY antigen is considered to be common among species, the HY antibody derived from rats and mice can be applied to cattle. The HY antigen is expressed at early developmental stages. Sampling of a part of an embryo is not needed for sexing with the HY antibody. These are convenient aspects of the HY antibody method. However, the HY antigen is not a major histocompatibility antigen but a minor one, so that its immunogenicity is weak; it is difficult or even impossible to induce the antibody in some strains, or the titer of the HY antibody is low. Judgement of reaction for sexing is not always clear-cut. These drawbacks prevent this method from becoming a reliable sexing method.
3) Cytogenetic Method
   (1) Method Utilizing the Sex Chromatin
      By inactivating one X-chromosome of the two X's in mammalian females, dosage compensation is realized to equalize the gene dosage between males and females. The inactivated X is seen in the nucleus as a sex-chromatin. Since the inactivation occurs early in the development, the presence or absence of the sex-chromatin in a part of the trophectoderm microsurgically isolated from a blastocyst would allow prediction of the sex of the embryo. This method is partly successful in mice and rabbits, but cytoplasmic particles obstruct the observation of the sex-chromatin in other domestic animals. This method is not practically used.
   (2) Identification of Sex Chromosomes
      Instead of detection of the sex-chromatin as described above, direct identification of X,X-chromosomes or X,Y-chromosomes provides a reliable sexing method. Practically, an embryo is bisected and one half is used for embryo transfer and the other, for cytogenetic analysis. Fortunately, all 58 autosomes of cattle are V-shaped telocentric, the X-chromosome is large and submetacentric, and the Y-chromosome is small and submetacentric. When sample specimens are good, identification of sex chromosomes is easy. However, good metaphase spreads are not always obtainable, so that sexing ratios are reduced, limiting the practical utility of this method.

### Problems to Be Solved by the Present Invention

As the DNA polymerase chain reaction (PCR) (Science, 239, pp487-491, 1988) is now well established, attention has been paid to development of a simple and reliable sexing method using Y-chromosome specific DNA probes, which could overcome the drawbacks of previous methods, as described above.

Since a part of a template DNA can be amplified millions of times, sexing of embryos would be possible if Y-chromosome specific sequences could be demonstrated by PCR in a small sample from the embryos. The problems are how to get appropriate Y-chromosome-specific sequences, what primers to use for amplification, and the actual procedure of the PCR.

Sequences used in the present invention should meet the following conditions.
(1) Sequences on the Pseudoautosomal Region Are Not Used.
   Crossing-over occurs between the X- and Y-chromosome at meiosis. The pseudoautosomal region in which genes such as MIC2 are located (P.J. Goodfellow et al., Science, 234, 741-743, 1986) shows 99% homology between the X- and Y-chromosome. Accordingly, discrimination of males and females is difficult; primers from this region are not suitable for the present invention.
(2) Not all the Y-Chromosome Specific DNAs Are Effective.
   Zfy gene (D.C. Page et al., Cell, 51, 1091-1104, 1987), for example, is a unique sequence on the Y-chromosome. However, there is a quite similar sequence on the X-chromosome, too. Genes like this are difficult to use for sexing, because there is the possibility that almost the same length of DNAs will be amplified from male and female embryos.
(3) A Unique Sequence, Even If It Is Y-Chromosome-Specific, Is Disadvantageous.
   Sry gene (A.H. Sinclair et al., Nature, 346, 240-244, 1990), for example, is uniquely located on the Y-chromosome and there are no similar genes on the X-chromosome and autosomes. This fact eliminates the problem mentioned above in (2). In these cases, including case (2) above, however, a single sequence of 200-300 bases has to be amplified from approximately 3,000,000,000 bases of a genome. This is not efficient. A tiny contaminant may lead to failure. These unique sequences therefore are disadvantageous for sexing by means of PCR.
(4) Y-Chromosome-Specific, Repeated Sequences Are Suitable.
   If sequences repeated hundreds of, thousands of times or more on the Y-chromosome are available, these would be most suitable due to the principle of the PCR.
(5) Gender-Neutral Primer Is Also Needed as an Internal Control.
   Embryos from which Y-chromosome-specific DNAs are detected are males and the others are females theoretically. However, since PCR is supersensitive, the possibility exists that male samples do not show male signals, or female samples give male signals because of a minute contamination or a slight deviation of the experimental conditions. To verify sure experimental results, internal control primers which give a gender-neutral signal are needed so that male samples give both male-specific and gender-neutral signals and female ones, only a gender-neural signal. Repeated DNA sequences are also desirable as internal controls.

### Means to Solve the Problems

After intensive trials to solve the problems mentioned above, PCR primers meeting all the requirements described above were obtained and, by using these primers, a reliable and rapid method to sex bovine embryos was established in the present invention.

That is, this invention provides a sexing method of bovine embryos characterized by DNA primers that specifically hybridize to bovine male genome, DNA primers that gender-neutrally hybridize to both male and female genomes, isolation of a small amount of cellular samples especially from blastocysts for preparation of template DNAs, amplification of specific DNA sequences by the PCR, and identification of the PCR products for sexing.

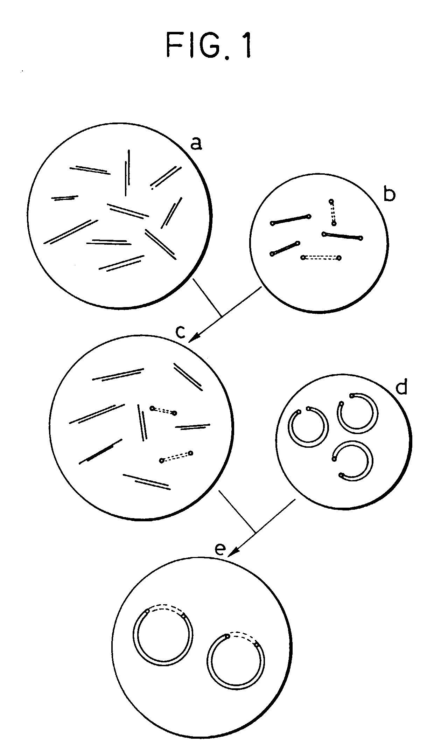

This invention necessitated preparation of DNA probes having the characteristics described in (1)-(5) above. The methods of preparation are as follows:
1) Isolation of Bovine Male-Specific DNA Clones
   Bovine female DNA is isolated as usual. DNA fragments without specific end sequences are prepared. The lengths of the fragments are preferably around 1000 bases. These fragments could be obtained by, for example, the ultrasonic treatment of the above female DNA.
   In addition, DNA fragments with specific ends are prepared by digesting bovine male DNA isolated as usual with a restriction enzyme of type II.
   The male and female DNA fragments thus prepared are mixed and subjected to the reassociation reaction, before which the DNAs have been denatured to single-stranded DNAs. The mixing ratio of the two DNAs is selected to give an excess of female DNA, e.g., 20 to 1000-fold. A desirable ratio is 50:1. To denature double stranded DNAs to single-stranded DNA, various methods, e.g., heat treatment and alkaline treatment, can be used. The reassociation reaction can be done by any established method. The method employing phenol, which accelerates the reassociation, is favorable.
   Since X-chromosomal and autosomal male DNAs are common to female DNAs, after the association, most male DNAs hybridize to complementary female DNAs which exist in excess. Because these hybrid molecules consist of female DNAs without specific ends and male DNAs with specific ends produced by digestion with a restriction enzyme of type II, the ends of the hybrid DNAs do not match. On the other hand, Y-chromosome-specific male DNAs cannot find complementary strands of female DNAs and hybridize to their partners to form the original double stranded DNAs with specific ends. When double-stranded DNAs thus obtained are ligated into cloning vector DNA with specific ends, the male-specific DNAs are selectively cloned into the vector DNAs. This cloning process is shown in Fig. 1.
   The recombinant cloning vectors described above are transfected into a host and, after confirming that bovine DNAs have been introduced into the vectors by using appropriate markers, clones containing the desired DNAs are isolated.
   Markers available here depend on the vectors and include resistance to antibiotics such as ampicillin and tetracyclins, and hydrolytic activity towards X-gal, a dye. Isolation of clones with expected DNAs can be done either by Southern blot hybridization, where plasmid DNA extracted from each colony is radioactively labelled by a well-known method and is hybridized to bovine male DNA, or by the plaque hybridization method when the cloning vectors are phages. Sequences of thus cloned DNAs can be determined by a well-known method, e.g., Sanger's method.
   For isolation of different clones specific to bovine males, screening of bovine male DNA libraries with male-specific DNA fragments as described above is conducted. DNA sequences of these secondary clones can also be determined by a well-known method as mentioned above.
2) Isolation of DNA Clone Which Gives Differential Southern Blot Images between Bovine Males and Females.
   By screening bovine genome libraries with DNA probes obtained previously as described in 1), many clones can be isolated.
   Most of these clones contain bovine Y-chromosome DNAs of 15-20 kb. These long sequences sometimes contain DNAs hybridizable to the X-chromosome and/ or autosomes in addition to Y-chromosome-specific DNAs.
   When DNAs are isolated from these clones, purified, and then used as probes for Southern blot hybridization, differences in electrophoretic patterns such as the number of bands and the intensity in the autoradiogram are observed between males and females. There are two ways to obtain PCR primers for sexing from these clones. Firstly, DNAs are digested to smaller fragments by using restriction enzymes or by some other method, and then fragments which hybridize specifically to males and gender-neutral fragments are subcloned. Secondly, after determining all the sequences, PCR primers are randomly synthesized and PCR products are examined to see if they are male-specific or gender-neutral.
3) Isolation of DNA Fragments Which Hybridize to Both Male and Female DNAs
   As shown above in 2), gender-neutral fragments can be extracted from DNAs which show differential Southern blot images between males and females, either by sub-fractionation or examination of primers which give gender-neutral PCR products.
   On the other hand, the Y-chromosome contains many DNA fragments which hybridize to both the X-chromosome and autosomes. Therefore, it is probable that gender-neutral fragments are isolated at the same time during the process of cloning of male-specific DNAs as described in 1). Since the sequences of these DNAs, once cloned, can be determined by a well-known method, these clones are useful to make gender-neutral primers.
4) Preparation of PCR Primers
   Parts of DNA sequences and their complementary sequences which hybridize male-specifically or gender-neutrally obtained as described above in 1), 2) and/or 3) can be used as PCR primers. The length of primers is usually around 10-40mer and a practically convenient length is 15-25mer. Primers can be prepared by digesting DNAs obtained as described above in 1), 2), and 3) with restriction enzymes, denaturing the fragments, and purifying single-stranded DNAs. Primers can be synthesized with a DNA synthesizer. Simplicity and reliability of preparation make the method of DNA synthesis preferable.

Next, the method of sexing of bovine embryos is explained.

Target embryos for sexing should be developed sufficiently that sampling of cells does not cause critical damage to them, but not over-developed, so that embryos cultured *in vitro* show no degeneration. From this point of view, use of blastocysts, in which the trophectoderm and inner cell mass have been developed, is desirable.

Sampling of cells from blastocysts is performed by a well-known method. First, embryos are transferred from culture medium into an appropriate physiological fluid such as Dulbecco's modified phosphate-buffered saline.

Sampling should be done after blastocysts have become attached to the surface of the dish firmly because of reliablity. Micro-blades can be used for sampling. Parts of embryos to be sampled should be selected so that damages to embryos is as small as possible, e.g., a part of the trophectoderm. Ten cells are sufficient for sampling.

DNAs can be extracted from these cells by a well-known method and are used as templates for PCR, where two pairs of primers, male-specific and gender-neutral, are utilized for sexing.

When the PCR products are analyzed by gel electrophoresis and stained with a fluorescent dye, male embryos give two bands, male-specific and gender-neutral, and female ones, a single band, gender-neutral, thus enabling reliable sexing of embryos.

Primers for the PCR described above have to be selected carefully so that the lengths of PCR products are sufficiently different to allow clear differentiation of the male-specific sequence from the gender-neutral one and so that the lengths of the products are not too long, but are compatible with the activity of DNA polymerase. Practically, the difference of the lengths of PCR products is at least 20 bases and desirably, around 50-100 bases. The length of each product are restricted to 100-500 bases and desirably, 150-300 bases.

### Effects of the Present Invention

The present invention provides PCR primers for simple sexing of bovine embryos and a reliable and rapid method for the sexing by the PCR technique.

### Embodiments of the Present Invention

The present invention is explained concretely by means of Examples.

### Example 1

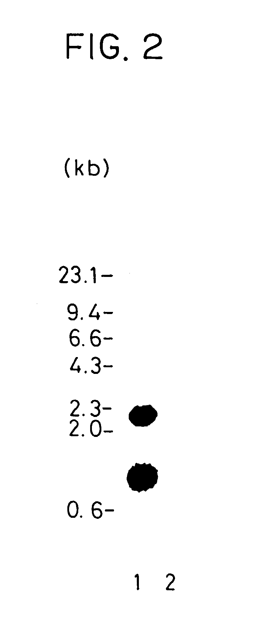

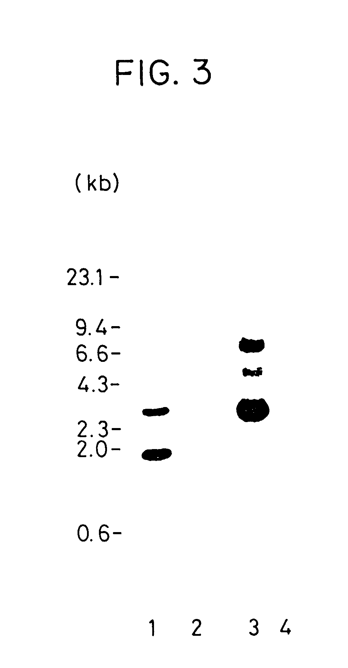

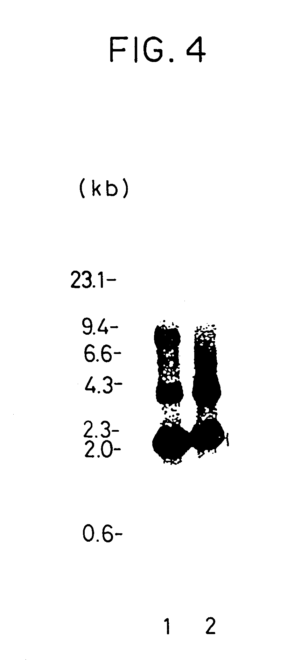

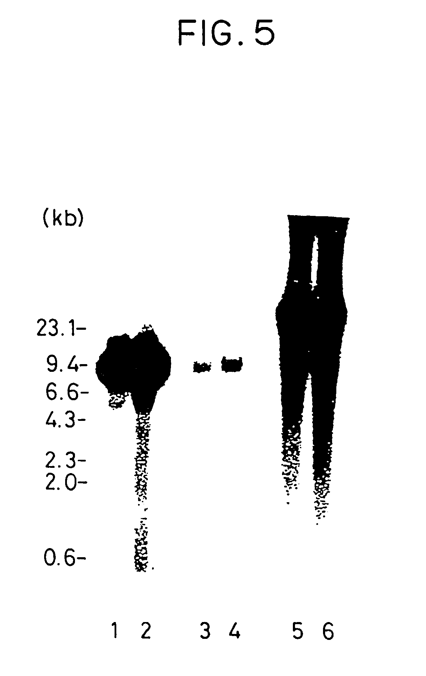

(1) Isolation of a Clone Having DNA Which Hybridizes Specifically to Bovine Male DNA
   DNA was prepared from the liver of a bovine female (Holstein) by the method of phenol extraction (J. Sambrook et al., Molecular Cloning, A laboratory Manual, 2nd ed., Cold Spring Harbor Laboratory Press, 1989). The DNA (200 µg) was sonicated to give fragments of around 1000 base pairs.
   Bovine male DNA (Holstein) was also prepared as described above and 20 µg of it was digested completely with 100 units of a type II restriction enzyme, Mbol (Takara Shuzo Co., Ltd), which cuts DNA at ↓ GACT sites.
   Female DNA fragments (50 µg) and male ones (1 µg) thus prepared were dissolved in 250 µl of distilled water and heated to 100 °C to denature the double-stranded DNAs into single-stranded DNAs. The solution was made up to 1 ml by adding 1 M phosphate buffer (pH 6.8, 120 µl), 5 M NaClO₄ (250 µl), phenol (81 µl), and distilled water (299 µl) and was shaken for 93 h at room temperature. After phenol extraction, DNA was precipitated from the aqueous phase with ethanol and dissolved in 20 µl of Tris-EDTA buffer. An aliquot (1 µl) was mixed with 200 µg of a plasmid vector pUC118 (Takara), which had been digested with a restriction enzyme BamHI (cutting site: G ↓ GATCC, Takara) and of which the 5'-phosphate residues had been removed. The mixture was treated with a ligation kit (Takara) at 16 °C for 2 h to obtain recombinant plasmids with insert DNAs having GACT ends.
   The reaction mixture described above was used directly to transfect *E. coli* DH 5α (obtained from the Institute of Applied Microbiology, The University of Tokyo) by a well-known method. The transfectants were plated on LB agar plates containing X-gal (40 µg/ml), IPTG (40 µg/ml), and ampicillin (50 µg/ml). After incubation, 400 white colonies, i.e., recombinants lacking the ability to hydrolyze X-gal, were isolated. DNA was extracted from each colony by a well-known method and was examined to see if it was derived from the bovine Y-chromosome DNA. For this, each plasmid DNA was labelled with ³²P by the random primer method and was hybridized to male and female DNAs, 10 µg of each of which had been completely digested with 100 units of EcoRI (Takara) at 37 °C, gel-electrophoresed, and transferred to a nylon membrane. The presence of bovine male-specific DNA in each plasmid was examined by making an autoradiogram.
   The result showed that a plasmid DNA specifically hybridized to bovine male DNA (Fig. 2, lane 1. Lane 2 shows the result with bovine female DNA). The sequence of this DNA determined by Sanger's method is shown in Sequence No. 1. The plasmid having this DNA sequence was transfected into *E. coli* by a well-known method and the transfectant E.c.118-bms1 has been deposited as FERM BP-4095 in the Fermentation Research Institute, The Agency of Industrial Science and Technologym, the Ministry of International Trade and Industry.
   For security, other clones were sought. The Sequence No. 1 mentioned above was used as a probe to screen a bovine male genomic library, which had been made by a well-known method, by recombining the EMBL3 phage vector (Stratagene) with partially Mbol-digested DNA. The plaque hybridization of 250,000 from the library consisting of 500,000 clones gave 28 positive clones hybridizing to the probe. One of them was picked up and its DNA was extracted. The DNA was completely digested with EcoRI at 37 °C. Each EcoRI fragment was examined by Southern hybridization for male specificity. The sequences of DNA fragments thus obtained were determined by Sanger's method to be as shown in Sequence No. 2 and No. 3.
   The results of the Southern blot analyses using DNA sequences shown in Sequence No. 2 and No. 3 are presented in Fig. 3, where lanes 1 and 2 are the results with Sequence No. 2 and lanes 3 and 4, with Sequence No. 3. Lanes 1 and 3 are hybridization patterns with male DNA and lanes 2 and 4, those with female DNA.
   These results demonstrated that both DNAs shown in Sequence No. 2 and No. 3 specifically hybridize to the male DNA.
   The original phage was one of 28 clones selected from 250,000 plaques, indicating approximately one positive clone per 10,000 plaques. The intensity of the Southern blot images suggests that each clone may contain 10-100 copies. On the other hand, to screen out a gene which exists only once in the human genome usually requires 1,000,000 plaques. Roughly speaking, the copy number of the original clone may be 1,000-10,000 in the genome. Accordingly, secondary clones can be quite easily isolated if one uses DNAs shown in Sequence No. 1, No. 2, or No. 3 as probes. PCR products are amplified approximately twice per PCR reaction cycle. One thousand is nearly 2¹⁰ and10,000 is 2¹³. Theoretically, therefore the number of PCR reaction cycles for this multi-copy DNA can be reduced by 10-13 times as compared with amplification of a single copy gene.
   DNAs shown in Sequence No. 2 and No. 3 were introduced into *E. coli* by a well-known method and the resultant recombinants E.c.gem-bms1 and E.c.gem-bms2 have been deposited as FERM **BP-4089** and FERM **BP-4090** respectively, in the Fermentation Research Institute, The Agency of Industrial Science and Technology, the Ministry of International Trade and Industry.
(2) Isolation of DNA Clone Which Gives Differential Southern Blot Images between Bovine Male and Female DNA.
   One clone was selected from the 28 clones described above in (1) and its DNA was extracted. One of the EcoRI fragments was isolated and sequenced. The result is shown in Sequence No. 4. This EcoRI fragment, consisting of 2104 base pairs, was labelled with ³²P and was used as a probe for Southern blot analysis. The result is shown in Fig. 4, where lane 1 is the hybridization pattern with male DNA and lane 2, that with female DNA. DNA of Sequence No. 4 hybridized to both male and female DNA, but images were different from each other.
   DNA having Sequence No. 4 was introduced into *E. coli* by a well-known method and the recombinant E.c.gem-bms3 has been deposited as FERM **BP-4091** in the Fermentation Research Institute, The Agency of Industrial Science and Technology, the Ministry of International Trade and Industry.
(3) Isolation of DNA Clones Which Hybridize to Both Bovine Male and Female DNA Gender-Neutrally
   When male-specific clones were explored as described in Example 1, 20 clones were found to hybridize to both male and female DNA in the Southern blot analysis. These were utilized for the present purpose. Taking into consideration the intensity of hybridization images, three clones which were expected to be repetitious were selected. As was done above in (1) and (2), sequences of EcoRI-HindllI fragments of these clone DNAs were determined and are shown in Sequence No. 5, No. 6, and No. 7. These EcoRI-HindIII fragments were labelled with ³²P and used as probes for the Southern blot analyses. The results are shown in Fig. 5.

In Fig. 5, lanes 1 and 2, lanes 3 and 4. and lanes 5 and 6 show the results of the Southern blot analyses with ³²P-labelled probes of Sequence No. 5, No. 6 and No. 7, respectively. EcoRI-digested male DNAs were applied to lanes 1, 3, and 5 and EcoRI-digested female DNAs, to lanes 2, 4, and 6. These results indicate that the copy number of DNAs shown in Sequence No. 5 and No. 7 is quite large.

DNAs consisting of Sequence No. 5, No. 6, and No. 7 were introduced into *E. coli* and the resultant recombinants, E.c.118-bmf1, E.c.118-bmf2, and E.c.118-bmf3, have been deposited as FERM **BP-4092** FERM **BP-4093** and FERM BP-4094 respectively, in the Fermentation Research Institute, The Agency of Industrial Science and Technology, the Ministry of International Trade and Industry.

### Example 2

### Synthesis of Primers for the PCR

(1) PCR Primers which gave male-specific bands were synthesized with a DNA synthesizer (ABI) on the basis of Sequence No. 1, No. 2, No. 3, and No.4. Sequences of these primers are shown in Sequence No. 8, No. 9, No. 12, No. 13, No. 14, No. 15, No., 16, No. 17, No. 18, and No. 19.
   Among these, combinations of primer Nos. 9 and 13, Nos. 8 and 13, Nos. 8 and 9, Nos.14 and 15, Nos. 16 and 17, and Nos. 18 and 19 gave male-specific PCR products. With the combination of Nos. 12 and 13, however, the PCR did not proceed.
(2) PCR primers which gave gender-neutral bands were synthesized as described above on the basis of Sequence No. 5, No. 6, and No. 7.
   Sequences of these primers are shown in Sequence No. 10, No. 11, No. 20, No. 21, No., 23, No. 24, No. 25, No. 26, and No. 27.
   Among these, combinations of primer Nos. 10 and 11, Nos. 20 and 21, Nos. 22 and 23, Nos. 24 and 25, and Nos. 26 and 27 gave gender-neutral PCR products.

### Example 3

### Sex Discrimination with Bovine Male and Female DNA

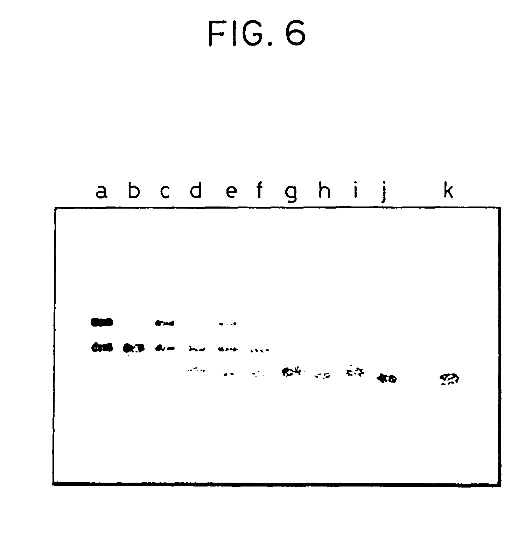

DNAs were amplified by 50 cycles of PCR with varied amounts of purified bovine male and female DNAs and primers of Sequence Nos. 8, 9, 10, and 11 (20 pmol each). The products were electrophoresed in agarose gel, stained with ethidium bromide, and photographed under UV irradiation (Fig. 6). In Fig. 6, lanes a, c, e, g, and i represent male DNA and lanes b, d, f, h, and j, female DNA. DNA (1 ng) was applied to lanes a and b, 100 pg to lanes c and d. 10 pg to lanes e and f, 1 pg to lanes g and h, and 100 fg to lanes i and j. Distilled water, the negative control, was applied to lane k.

The results indicate that 10 pg of DNA is enough to discriminate males from females (Fig. 6, e). Since the DNA content in one cell is approximately 3 pg, sampling of three cells from a blastocyst should be suffice for sexing.

### Example 4

### Sex Discrimination with Cultured Bovine Cells

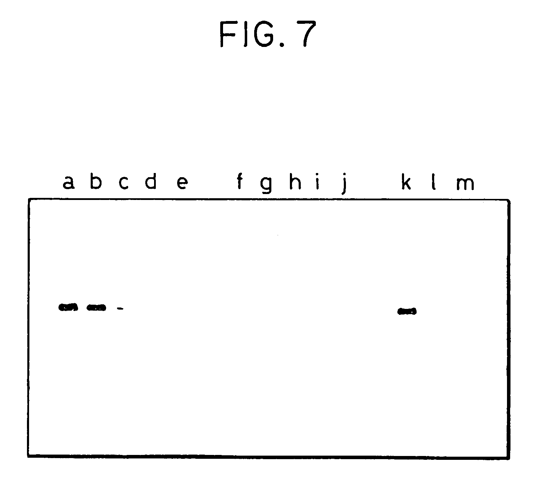

Bovine fibroblast-like cell cultures were obtained from the liver of a male and a female by a well-known method (The Japanese Tissue Culture Association (Ed.), Techniques for Tissue Cultures, 2nd Ed., Asakura Publishing Co., Tokyo, 1988, in Japanese). Cells were frozen in Tris buffer and then thawed for 30 min at 90 °C. The cell suspension was serially diluted and subjected to PCR for sexing with primers of Sequence Nos. 8 and 9 under the same conditions as described in the above Example 3. In Fig. 7, PCR products from male cells were applied to lanes a-e and those from female cells, to lanes f-j. The expected numbers of cells used were 100 for lanes a and f, 30 for lanes b and g, 10 for lanes c and h, 3 for lanes d and i, and 1 for lanes e and j. PCR products from purified male DNA(10 pg) as used in Example 3 were applied to lane k and those from female DNA (10pg), to lane 1. Lane k was PCR products of phosphate-buffered saline, the negative control.

The results show that 10 cells are enough for sexing.

### Example 5

### Sex Discrimination with Bovine Embryos (1)

Blastocysts cultured *in vitro* by a well-known method were put in Dulbecco's modified phosphate-buffered saline (D.G. Whittingham, Nature 233, 125-126, 1971) and, by leaving them at room temperature for about 10 min, allowed to become attached to the surface of an untreated, plastic culture dish.

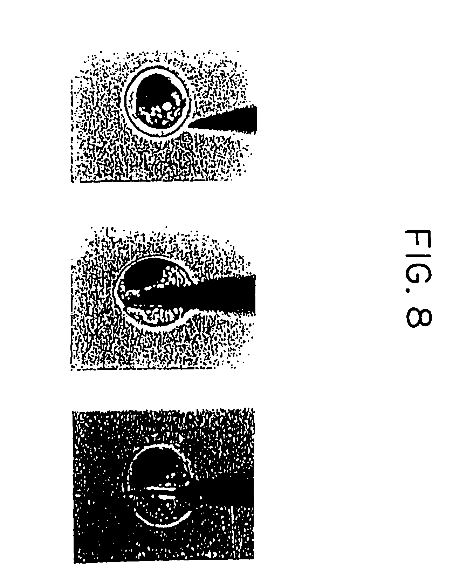

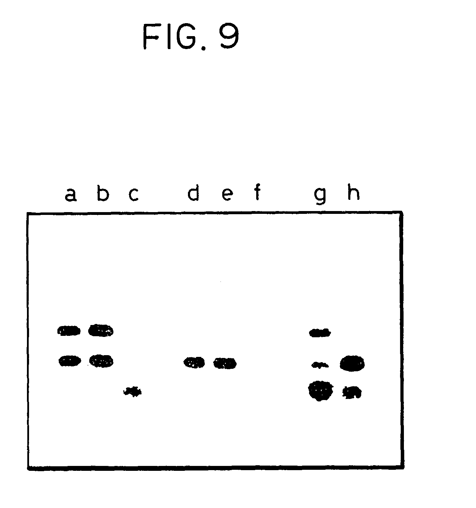

A part of the trophectoderm was removed the blastocysts by cutting with a microblade as shown in Fig. 8. The sample and remaining blastocyst were sexed by the method of the present invention with PCR primers of Sequence Nos. 8, 9, 10, and 11. This sexing method was the same as in Example 4. The results of this sexing are shown in Fig. 9, where lanes a and b represent results of the assymmetrically bisected samples from a single embryo, as do lanes d and e. The medium used to culture the embryos sexed here was used as the negative control and the PCR products were applied to lanes c and f. Lanes g and h are the positive controls, i.e., purified male and female DNA (10 pg each), respectively, as used in Example 4. As a result, both lanes a and b which were derived from the same embryo gave PCR products with the same lengths as the positive male DNA, implying that the original embryo was male. By the same token, the embryo used for lanes c and d was female, because PCR products were the same as the positive female DNA.

### Example 6

### Sex Discrimination with Bovine Embryos (2)

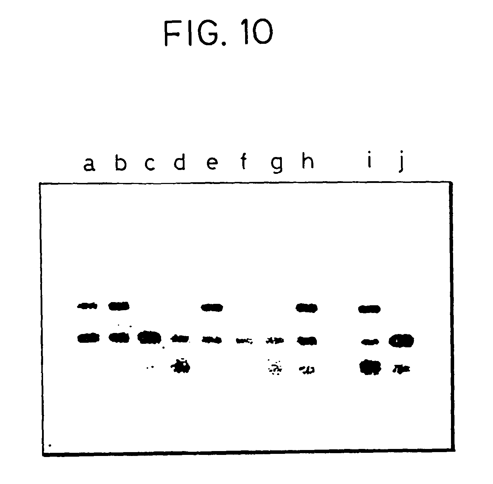

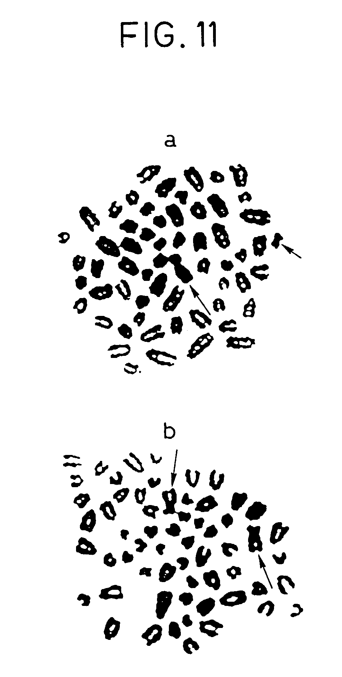

Blastocysts were bisected to obtain 8 pairs of demi-embryos and each demi-embryo was subjected to sexing by the same method as described in the Exmple 5. The results are shown in Fig. 10, where lanes a-h are results of the PCR with demi-embryos. Lane i represents the result with purified male DNA (10 pg) as used in Example 3 and lane j, the result with female DNA (10 pg). As a result, embryos in lanes a, b, f, and h were judged to be males and those in lanes c, d, and g, to be females. The other halves of the same embryos were cytogenetically examined under the microscope. Out of 8, karyotyping of samples corresponding to lanes c, e, and g were successful. The demi-embryo used for lane e was male, having the X- and Y-chromosome as shown in Fig. 11, a, and those used for lanes c and g were females, having two X-chromosomes as shown in Fig. 11, b. Demi-embryos for this karyotyping were cultured in TCM-199 medium (L. Leibfried and N.L. First, J. Anim. Sci., 53, 76-86, 1978) in the presence of Colcemid (0.04 µ g/ml) for 2 hr at 37 °C, treated with a hypotonic solution (0.9% sodium citrate) for a few minutes, fixed with a mixture of methanol: acetic acid:distilled water (3:2:1), applied to glass slides, stained with Giemsa's fluid, and then examined under the microscope. Long arrows indicate the X-chromosomes and the short arrow, the Y-chromosome in Fig. 11.

### SEQUENCE LISTING

Sequence No. 1
   (1) Length of sequence: 1404
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GATCCTCTCA CCTGTCCCAG TCCCACAGGC CTGGGCTGAT GGGGTAGGCT GGCGTGTGTT 60

CTCACCTGGG GGCTGGATGA GAACAGAGCT ACTTTGAAAG CGCCTCTGTA CAATAAATGG 120

GCAGACTTCA ATTCCTCTGG CACTGGTGAG TCATGGCTCT TAGCGGGACA CTGTTCTTAG 180

CATCTCCGGA TTGAGAGATG TTTCGTCTCT AAATCTCTCT CTACACAGAG AAGTCCAGGT 240

TTGTGTCACA TTTGGACACG TGGGCAACAG TCTTTTATGT GAAGCGCGGA ACTCGTTATT 300

TCCCGAAAGA ATACATGCAT TAAGTTTTCT GCCAAGAAAT GTGGCATTCT CTCTGTTTCA 360

CCTGTGAAAA TCTCACCACA AACTTCCCAA AAGGGAAATG GAATTCTCTA TATGTTATAT 420
 
GAATCATGGG TGCACGTGTT ATTTGCCGGC ATGAAATTTG GAATTCTGTG TATTTCCCGT 480
ATGAAAACTT GGGTGCTGAC TTTTGCACAT GAAATCTGGA ATGTCGTGTA TTAAACTTAG 540
GAACATCAGG CCACTGATTT TAGCACACAA AATACGGAAT TTTGATGACT TTACGTATGT 600
AATCCGGGGC ACTGATTTTT CCTCTGTTGA GTACAGATTT CCTTACATGT CTTTATCTTT 660
CCTCTGTAAG AAAATCGTGG CAGAGACTTA GTCCATGAGC TAATGAACTG TTTATATTTC 720
ATGTAGGAGA ATCTGGGTCG TGATTTTCCT ACATGAAACA TGCAGTTCTC TGTATTTAAT 780
GAATGAACAT CCGGGCACCG ATTTTCCTGC CTTGAATATG GGTTTCTCTA CGTCGGCATT 840
TGAAACTCCA GGCACGGAGC TTTTCCCCGT GAAATATGAA ATTCTGGTAT TTCACGTTGA 900
ATTGGGCATA AACAAGGTAA CACGAAATAC GGAATTTTTC ATCTTTCTCA TTTGAAAATC 960
TGTGTGCTGA CTTGCGTTAT GAGTTACAGA ACACTGTATA TGTAACAACT GAAAGAATTG 1020
GCCTCGATTT TCCTACACAA AATATGGAAT TTTCTATTTC ATACATAAAG TGATTTTCAC 1080
ACTTGAGGTA TGAAGTCACT GTCAATGAAA ACACGAAGTC AATCCTTGTT CAAGGTGATG 1140
AACAGGTAAG TCAAAAACCT TGAGCATGTG ACCACACCTG AAAGGGAACC CTGAATGTTC 1200
TGAAACTCTG AGACTGGATA AAACCTGGTC TTTTATGCAA AATGGGGGGA CAGAAGTACT 1260
AACTAAGAGC CCTAACCCCA AGACCTGGAT GTTTCCTCTT TAGCCACACT CACTCTTGAG 1320
CAGGGTTGCT TTAAAAAGCC ACATGTAAGA ACAATCAGTC TGGGGACAGC GGACAGCCGT 1380
GGTCAGCCGG CCTGGTGTGA ATTC 1404
Sequence No. 2
   (1) Length of sequence: 1655
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GAATTCGTGA GATGCAGATC TAGACGTGCT TATAATTTTA AATGCTTGTA TTAGAAAAGA 60
ATAAAAGCCT AAGCCAGTGG AAAGAGAAAT GGCAAACTCC GTCAAGGCTT TCAGCAAAGG 120
CCACATAGAG GAAACGTGAG GGAGGGGTGT GATTAGCCGG TGCAAACTTC TTGCTGTCAC 180
ATCTTGTGTT CTTGAGGTGA AGTGGTGGCC AGCTAACTGT GTCCTGCAAT CCTCAAGGGA 240
AACAAGTATG ATTCCCTATC CCCTGAAAGA GATTCCCCAG ATTGACTTTC ACCCTCCAAG 300
GTCCTGGTTG CGAGAAGGGG GTCCCCGTAT AGCCTGGTAG TCCCAGCTGG AAGAGGCAGG 360
TCTCAGTTCT CTGTGCCCTC CTCTTACCAA GGCCCCCACA CACTGCCCGG CCACCTGCCA 420
TGAGGGAGCC AGGTACCCAG CACGCAGCTG ACCCTCAGCC TCCTCCGTCT ACCCAAATGG 480
GGAGCTCGGT CCTGTAGACT GTGGCCCATG GACATTGCCA CAACCATTCG GACATGGAGG 540
TGGGCATGGG ACACGGTGGC CGTTGCTTTG AAACCTGGGC CAGGCCGCTG AGTGGCTCCA 600
GCAAGGCCTC CGGGACTCTG CAAGACACAG CCTTTCCTCA CGCTTGTCCA AGCACCACAG 660
TTCATTACCC AGCCCAAGGC CCAAGGCTGG AAGGCCTGTG GGAAGGCTTT GATATCTCTC 720
TCAGTGCATT CAGCTGAGAG GCTTTCTGTT GGTGGTGTTG GGTTTGCGAA CTTCCCTAAT 780
GGTCTTGGCT GAGCTGCAGC TCCGTTTTCC ATCCTGCCTC TTTTACAGTG TCTTCCTGGA 840
TGAGCTGAGT CAACCTGTGA CTAAAAGATG GTAACGTGGA CCATCGGGGG CCATTTCCAC 900
TGGCCCGATG ACCACATCCT ACTCTCTTTC TATAACGCAG GGCTTTCAAC GTGACAATGT 960
CGTGTGATGT AAAAGCCTTC ATCTTCCTCG AACTCAAGTA TTTAACTCAG CATACTCTCC 1020
TCAAGAACAT GTATTGCATA CAGGGCTACC ATTTATTTTT TCACTGCCGG AATTATACTG 1080
GACATGTGAT GTACTTCCAA CCATTAATTT GGGGCCATAG TCACGTTACA GTGTTTCTTT 1140
GGTTTCTGCT GTGCGTGAAT CAAGTTTTAG CTGTACGTGT ATCCCGTCAA TTTTGGATTT 1200
CCTTCCCATT TAGGCCACCA CAGAGCAGTC GAGTCCCTTT CTTCTTTCAG TTTTTCTTTT 1260
TATTAAATTC AACTGGGGGT TAATTATGAT ACAATATTGT AATGGCTTCT GCCATGCATC 1320
AACATTAATA AGCCATAAGT ACACAGGGGT CCCAGCTATC CTGGAGCCGC TCCCACATCC 1380
CTGCCTACGC TATTCTTCGG GATTGCTCCA GAGCACTTGC TCTGCATTCC CTGATTCATG 1440
CAGATGAAAA CCAGAAAGAT GATTTTTCTG CATAAAATAT GCAATTGTCC TTCTTTCACA 1500
TGTGAGAACA TGGGTGCAGA GGTTTCTACC TGAAATAGGA AATCGGTATT TCATGTATGA 1560
AAATCGACTT GCATCATTTT CTTCATCATA TGTGGAATCT TGTGTCTCCC AGACGTATAT 1620
CTGTGCACAC GTTTTCCTAC ATGAGTGGGG AATTC 1655
Sequence No. 3
   (1) Length of senquence: 3068
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GAATTCCTCC ACCTGCCAGG ACAAAGTGTG CGGGGTGGGG TGGGTGTGAA ACCTCTTTAC 60
AGAAGAGCTG TCCTTTCCTG TGTTAGGGAT AGACCATGCC CTCTCCCCTC CCCAGTCACC 120
TCCCCTCCAC AATCATCAGT GTCCCCTGCC TGACCTCCAA GTTGGTCATG AAGTGAAGCA 180
TGTCTGCATC TTGCTTGCTC ATCAAAACTG ACATTTGGGG GTGGTTCATA AACTGCTTTA 240
GGTCAAGGAG CCTCTAGATG CTAGAGGTAA AAGTGCTGGA AGAACAAAGC TAGCCTAAAG 300
AGTAGAATGG CCCTCCCTGT TTGACTTCAA CTTGCTACTG GTTAACTCGT CACATTTCGG 360
TTCACGCGGG GCTATATGAA TGCCGCACAA GGTCCAAGCT GTGCCCATGA ATGCCCTACC 420
ACGAGGAGGT TCTCTCCTAA CCGGATAAGC TTCATCTCAG CCCCTTGAAA GTTAGCTTAC 480
TTCGGGAAAA CAAGCACTCC TAGCTAGAAC CCGTAGACCA CTTACACTTC CCCACCCCTC 540
CCCCATTCCA GACAAGCCTT CTCTCCGAAG AGACCCTGGT GCTAATGACA ATTCTGATAG 600
GGACCTCTCA AAGTATAGCC CAACTATCAA TTTGGGCTAG CCAGAACAGC AAGGACATCA 660
CACCTACGAT CAGGAACAGA TATGCAGGAC GGTAACCAAT ACCCACCATC CAGAAAAGTA 720
AATACGGTAC CAGAACTGCC AGATCTGTAA ACTGTTCCTG CCATAGCCCG GCTTAAACAA 780
ACAAACAAAC ACAAAACTCA CAAGCACCAC ACCAAGGGAT ACAACTTCGA CCCAGAAGCC 840
ACGGATGCCC TGGATGATGG CGCTTCTGTG CTCTAGATGC ACCTTCCGCC GCTGACTGGT 900
CTTATGTTTC AGGCGAGCAT GCGCCTTTCG GCTTTCTTAT TCACGGGCTC CAGCTCCAGC 960
TGCAGGGCCG CCAGCGCTCC AGTGCAGGCC GGTCACTCAG GGCTCCGGGC CTTGGATGGT 1020
CGTGGACATG CTCCTCCGAG GACACCTGCT CCTGCTCCTC GTATGCCACC ACCTCCACCT 1080
 
CCTCCATGAC GTCCAACACC AGCAGCTGGA ACTCCTGCCC GAGTCCCGCC ACCTCTCCCT 1140
CCTCCACAGC CGCACCTTCC TCCACTGCCT CCACCCAGAA GAGGGCGGCC TCCTCGCCTG 1200
GCGCACCACC TGCGGCCTGC ATCGCCTCTG CCGCCCCCAC CGGACTGGAA GGCCAGGGCC 1260
CCTCCCTCAT GAAGACCCGG GCTCACAACT AAGATCCAGG ATCCCGGAGT CCTGCCGCCT 1320
TCCTCTGGCC CCGTCTCACT CTCCATGGTA TTCTCGCCGA AGCCCGGAAC TGCAAACAAG 1380
CTGGACGCTA GAGCACGGCA GACGGCCCTC ACGGCCCGCC GGCGCAGTCT ACGTGGTCCT 1440
ACTCCCGACG GTTACTGGGC AAGAGCCAGG GAACGGCGAA AGGGAGAGAC GCATGCGCAG 1500
AACCCTCTGC CACCAGGCCG CCTACTATGG CGACGGGAAG GGGCTGAACT CTCCACCCTG 1560
ACCTCCTGAC CTCATCCCAG AACCAATCAA ATGGAGTCTA AAGCGGTTCG CTCCTGGACA 1620
CGCCCCTTGG AATCCTGGGC CCTCTTGCCA CCTGTGACAG GCGCCAATGT TGGCCCAGCG 1680
CAAAGTGGGC GTGGCATGCA AGCCTTTTGC CTGCCTAGCA GTGCAGCCGC GCCCGAGCAG 1740
CGACTGGGAG GCCAGGGCCA CCTGAAGCTG CAACAGTCCT CAAGCTTGAA GTTGCCCCTG 1800
GGGCAGCGCG CCCGTGTGCT CAGGGACACA CTCAGGAAGA CAGGGTTCCT GGGTACCTCA 1860
GGGACAGAGT TGCTCTCCTC CGATCCAAAC CGCAAGCCAC GGGGTGGGGT GGGGGGAGTA 1920
GGAGGGAGGC GGGCAGGGTA GGGGGGTTTG GGGGAGGAGT GCGCGGTGGA GGGGGATGGG 1980
GAAGCGGGGC GGGGCGGTGG GGGCGGGCTT ACGCGACCTC ACCCATGTGC ACCTGCTGCA 2040
GAGTCATGGC TAAACTTGTG CCTTAGGTTG AGCAGTTGTC AGGAATGCAG CCCTCCTCTG 2100
AGGAAGATGC AGAGCACTTT CCTCGGCTAG CTGTCCAAGC CCCACGATGC AGCCGCAAGC 2160
CCAACCAGCC TGGAGCCCCT GGCTACGATC TGTCAGCCCT CGGCTTTCCC CCGGCCCGTG 2220
GTTAGGGCAG GCGCCACGTA GCTAAGGATG TGCGCCTGCA GATGCACTTG CCAGAGGCAG 2280
AGCTATTCCT GCAAGTGTTT CAAGTGGACC AAAGTGCTCC TCCTGACCAT TAGTTACTCC 2340
TACGTCCGGG CCGCCTAGCT CAGGTGGGCA CACTCAGGTT AAGTGCCTGG ACAGTTTCCA 2400
CTGCTCACGC TCTTTCTCCT AGGATGTCTG CTGTTTCACA CTGAGCACCT ACCTCCCTTA 2460
GTCCTGACAC ACTCACAGGG CCACCACAGC CCACCCCACC CCCCACACCC CCCCACAAAC 2520
ACACACAAAC ACGTGACACA AGTACACACA TGCGTGCAAC ACACAGAGCC ACAAATGCCA 2580
ACAGGTGTTT CAGAGACTGA AGAACCAGTG GTTCGCATGA AATGATAGAT GTCTTTATTT 2640
CTAACGGGAA AATGTTCATT CTGATCTTCA GATGTGAAAT ATAGAGAAGT CCATCTTCCA 2700
TGAGGGAGAA TCATGCCTAG ATTTGGATAC AGGAAGTATA GAGGAACTCC TTATTTCATG 2760
GTGGACAGTC CTTGATTTAT GCATGAGAAA TACAAGGAGT TAAGTATTTC ACGAAGAAAC 2820
 
AGTGGTGCCC AGAATTTCAT TTATGAAATA GAGATAATTC CAATTCCATA TTGGTTCACC 2880

AGATCTTAAT TTCACACTTC ATGTCAGTAA ACTTTCATTG AGATCTTCAT ATACGAAATG 2940

TATACAATTT CAGGATTCAA GAAGGGAATT GTAGGCTCAA CTTTTCATAT GTAAAATATA 3000

TAGAATTCCA AATTTCATGC CGGCAAATAA CACGTGCACC CATGATTCAT ATTACATATA 3060

GAGAATTC 3068
Sequence No. 4
   (1) Length of sequence: 2104
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GAATTCACTG CAAAGGAAAA ATAAAAAGCA TCAGCTGCAT GCGTGCGTGT ATTGTTTGGG 60
CAGGTCCTCA GGACAAACAC TTCAGCTGCA TGTACGAGTG TCAGTTCACC GCGTGGGAAT 120
TACCAGTCTG TGGAGATTCA TAGAGGGGGC TAAGTCGTAT GTTGGGGTTG TGTCAGGGGG 180
TGACGTTCTT CCTGTGGGGT GGGGGTTGGG GAGGACGGAG AAGGAAGATG AGGGGCCTAT 240
TGACTGTGGG TCTGGCAATT TTACAAGGGA AATGTGGCTG TTCGTGGAAA GGGTCTGGTG 300
AACAAGGGCC AGAGATTGAA ACAGTGGAAT GCAATTCTGT TGGAAGGTGG TGGCCCAGGT 360
ATCTTCTCTG TTGCCTATCA TCAGACACTT TAATAAGGGT ACAGAACAAA GGAAAAGCCT 420
TATCATTGAA CTCGCTATGA ACCTCGAAGG CCACATGTGT ATGAGTGTGT ATATGAGTGC 480
ACGGTCAGCT TTGCTGAGAG TCTGCTTCCC AGAGCTAGGG TTCCAGGTAA CATCTTAGGG 540
CATTGTGTAT AAGGTATATG GAGGGGGGCT GTGGCCTAGG GAGAAAGTGA GGGGTGGGAA 600
GGCAGTGAGG ATACTAGTTT CTGGCAAAGG TGACCAGGGG AAAAGAGAAA TTGCAAGGAA 660
TCAGATAAAG GAAAACAGAT CCCGTCGTAG ATGTTAGAGC TCATGTGAAC TCTGTTATTT 720
 
TTTCCAAGAA CTCAGCTTCG GTATAGCGTG TCCAGGGGCA GAAGTGAGGG GAAAGAGGCT 780
GTTAGGGTGA GCCTATGCAG CCATGTGGTC TGTGTCCCAT GCTGCTGCTG CTGCTGCTGC 840
TGCTGCAGCT CAGGGGTCAA TATCCCTTTT TCCAGGAAGG CTTAAGGTCT TTGCCCAAGG 900
CCCTCTGTCA GGCCAAGCTG TAGAAAAAGC ATTTTTTCTC ACCAGCCCTC TGGTGACAGT 960
TTCTCTTCCT TGTGGAATGG CTCAGCACAG AATGTTTTCA ACCACAAAGG AGCTGTCCTT 1020
CATCTAGGGC TTTGCACTAC TGTGTGGGCC AAGGGCAGTG GGAACAGCTG GAGCACATGG 1080
AGCTCACTGA GGGACTGGTC GGCACTTAGG GTGGTCCTTC TGAAGTAGAT GACTGCTTTC 1140
TGGAATCGCG TCCACCTCTG GATTGAGCAA AAGACAGACT GGCACAAATG GAGTAGGCAG 1200
AGTTCAACGT CTGTCCCCCT ACTGTTGTCC TCTCATCCCA GATAGAAGGG CTCCACCGTG 1260
GGCATGCCTG AGGGTGATGT CCCACATGAA GTTCTGTCTC GCATGAAGTT CTCACTCTAC 1320
AACTTTTCTC CCTGAAGCAG CATCTCTTTT GAGGGCAAAG GGTAAAGTTC ACAGGTTTTA 1380
TGGATGTCCT CTTCACGGTG CCCTGCATGA GATCTCCTGC CCAGGGCCAG CTTTTGCATT 1440
TGACCATGTA CACCCCAGCT TTGCCCCTTC TCCAGAGACG CATCTACAGC GCTAGAAGGC 1500
AAGGCTTGGC CGTCTCTGGG TGCTTTCTGA ACAAGCAGCC CAGGAGACAC TGGAAACATG 1560
TCTTCTTACT TTCTGAATCT GTCAACGTCC AAGAATCCGT GGGGCTCCGG CAGGCTGGTA 1620
CCTGTCTTTC CCAGTCCTAG TTCAAAACAA AACAAAGCAG AAACTATGGT GTGATAACTC 1680
GACAGCGGGT GTGAGATGGC ATACTGGGGC ATGGTTTTCG GGACCTTAGA CTGGGCTCAT 1740
TCTAGGGACT GCATGGCATT TACCCACATT CAGAAGTTTC CGTAAGGCAT TGCAAAGAAG 1800
TTAAAGACAA GAAAAGTTTA GGACAGGGTC CCCCAGTGCC TAATATTTCC TGCCAGTGCT 1860
TCAGGACCAG GGTTGGCATT AGCTCTAACC TGTCCTCGAG TGGAGTGCAG AGATAGTCTT 1920
CGTTGACTGG ATGGTCATCC TCTGGAGACT TGCCGTGACT TGTGTCCCAG GCTCTCTAGG 1980
GCTTTCACTT GGGCCCTAAA GGCTAGGTGC TCTTAGAGTT GACTGCACAC TTGGATGTGT 2040
CGTGTAAGGT TTTCTTCCGC CGAATATCGA TGCGTGTGAG ACTCGCCGGT TTAGTGATGA 2100
ATTC 2104
Sequence No. 5
   (1) Length of sequence: 305
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GATCTGTTGG AGGTTCAGGT ATTGAGATTT GTTAGAAACC ATGCTATTGC TAAAATGAAA 60

CCAATGTCGC CGATGCGGTT ATATAAGATT GCTTGTAGGC TGCTGTGTTT GCATCTGTCG 120

TCCGTATCAT CATCCGATGA GTAGATGATA TGATTCCGAC GCCTTCTCAG CCAATGAATA 180

GCTGGAAGAG GTTGTTTGCG GTTACAAGGA TGAGCATAGT ATGAGGAATA GGAGTAGGTA 240

TTTGAAGAAT TTGTTAATAT TGGGGTCTGA GTGTATATAT CATATTGAGA ATTCTATAAT 300

AGATC 305
Sequence No. 6
   (1) Length of sequence: 373
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GATCAAGCTA AAGAAATCTT CCTTATTTGA AAGGCCGAAA GACAAGCAAG CAAGCAAACA 60

AAACAAAAAC CCACATCCCT AAATCAAACT ATTGCCCTAG CGCAAAACAG AAGCAGATTT 120

CACAGCCCGA GGTCAGCCTA GCAGGCTTGA GAAAGAATGT TTAATTATAA ACTAAAACAA 180
 
CAGGATTCCA AAGGATAAGC GGTCCTGCAG GGAAATCTAT TCGTTTTGAT TTTTTCTCTC 240
TCAGTGTCCT GTCAAATAGA AGCCACTTGT AGAATCCGCT AAACATTGTT CTGCATTAGA 300
AATATTGCAT ATTGAGCGCA CACACACACA CCACACACAT ATACAGAGGA AAGACTTACT 360
CTTGGGAATG ATC 373
Sequence No. 7
   (1) Length of sequence: 241
   (2) Type of sequence: nucleic acid
   (3) Number of strands: double-stranded
   (4) Topology: linear
   (5) Kind of sequence: genomic DNA
   (6) Origin
      (a) Species: *Bos primigenius*
      (b) strain: Holstein
   (7) Characteristics of sequence
   (8) Sequence 
GATCAGTGCA TAATCAGCCA CATAATCAGT GCATGATAGC CACCTGACCA GTGCATGATC 60
CATCTTTTGT TTTCTCCTCT GAGGAAGAAG CCCAAGTTGC TAAGCACTCT ATTTCCGTTT 120
CTTGGCTCCT CCTCCTCCAA CTTTAACAGT TTGCTTAATC AATCCCCATT TGCTCCGAAG 180
TCTAGTTCTG CCACTTCAGC TTGCTCCATC TTCTCTGTTG ACCCACATGC ATATTAAGAT 240
C 241
Sequence No. 8
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 509-528 of Sequence No. 2
   (8) Sequence TGGACATTGC CACAACCATT 20
Sequence No. 9
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 715-734 of Sequence No. 2
   (8) Sequence GCTGAATGCA CTGAGAGAGA 20
Sequence No. 10
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 90-109 of Sequence No. 7
   (8) Sequence GCCCAAGTTG CTAAGCACTC 20
Sequence No. 11
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 172-191 of Sequence No. 7
   (8) Sequence GCAGAACTAG ACTTCGGAGC 20
Sequence No. 12
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 482-501 of Sequence No. 2
   (8) Sequence GAGCTCGGTC CTGTAGACTG 20
Sequence No. 13
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 645-664 of Sequence No. 2
   (8) Sequence TGAACTGTGG TGCTTGGACA 20
Sequence No. 14
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 120-139 of Sequence No. 4
   (8) Sequence TTACCAGTCT GTGGAGATTC 20
Sequence No. 15
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 520-539 of Sequence No. 4
   (8) Sequence CCTAAGATGT TACCTGGAAC 20
Sequence No. 16
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 1580-1599 of Sequence No. 4
   (8) Sequence TGTCAACGTC CAAGAATCCG 20
Sequence No. 17
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 1881-1900 of Sequence No. 4
   (8) Sequence CTCGAGGACA GGTTAGAGCT 20
Sequence No. 18
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 1671-1690 of Sequence No. 4
   (8) Sequence GTGATAACTG CGACAGCGGT 20
Sequence No. 19
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementray strand of No. 2081-2100 of Sequence No. 4
   (8) Sequence TCATCACTAA ACCGGCGAGT 20
Sequence No. 20
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 58-77 of Sequence No. 5
   (8) Sequence AAACCAATGT CGCCGATGCG 20
Sequence No. 21
   (1) Length of sequence: 20
   (2) Type of sequence: nucleic acid
   (3) Number of strands: single-stramded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 252-271 of Sequence No. 5
   (8) Sequence CTCAGACCCC AATATTAACA 20
Sequence No. 22
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 146-165 of Sequence No. 5
   (8) Sequence TGATATGATT CCGACGCCTT 20
Sequence No. 23
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 225-244 of Sequence No. 5
   (8) Sequence CAAATACCTA CTCCTATTCC 20
Sequence No. 24
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 71-90 of Sequence No. 6
   (8) Sequence CCACATCCCT AAATCAAACT 20
Sequence No. 25
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 253-272 of Sequence No. 6

   (8) Sequence CTACAAGTGG CTTCTATTTG 20
Sequence No. 26
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: No. 113-132 of Sequence No. 6
   (8) Sequence GCAGATTTCA CAGCCCGAGG 20
Sequence No. 27
   (1) Length of sequence: 20
   (2) Type of sequence: nucleic acid
   (3) Number of strands: single-stranded
   (4) Topology: linear
   (5) Kind of sequence: other nucleic acid, synthetic primer
   (6) Origin
      (a) Species: *Bos primigenius*
      (b) strain: Holstein
   (7) Characteristics of sequence: complementary strand of No. 195-214 of Sequence No. 6
   (8) Sequence

TTCCCTGCAG GACCGCTTAT 20

### BRIEF DESCRIPTION OF DRAWINGS

- Fig. 1.: Schematic illustration of cloning method for male-specific DNA fragments.
- Fig. 2.: Electrophoresis of a clone which specifically hybridizes to male DNA.
- Fig. 3.: Electrophoresis of secondary clones which specifically hybridize to male DNA.
- Fig. 4.: The Southern blot images (electrophoresis) of DNA which consists of two parts, of which one specifically hybridizes to male DNA and the other, gender-neutrally to both male and female DNA.
- Fig. 5.: The Southern blot images (electrophoresis) of clones which gender-neutrally hybridize to DNA.
- Fig. 6.: Sensitivity of discrimination of sexes by the present invention with purified male and female DNAs (electrophoresis).
- Fig. 7.: Sensitivity of discrimination of sexes by the present invention with cultured bovine cells (electrophoresis).
- Fig. 8.: Method to cut out a part of the trophectoderm after allowing a blastocyst to attach to the surface of a culture dish (morphology of the organism).
- Fig. 9.: Sexing of demi-embryos by the present invention (1) (electrophoresis).
- Fig. 10.: Sexing of asymmetrically bisected embryos by the present invention (2) (electrophoresis).
- Fig. 11.: Microscopic images of karyotypes of demi-embryos (morphology of the organism).

## Claims

1. A DNA sequence as shown in Sequence No. 1, 2 or 3 which hybridizes specifically to bovine male genomic DNA.

2. A DNA sequence as shown in Sequence No. 4 which comprises two parts, of which one hybridizes specifically to bovine male genomic DNA and the other commonly to both male and female DNA.

3. A DNA sequence partially or totally as shown in Sequence No. 1, 2, 3 or 4 obtainable from bovine male DNA by using DNA probes.

4. A DNA primer which comprises at least one part of DNA Sequence No. 1, 2, 3 or 4 or the complementary strands and hybridizes specifically to bovine male DNA.

5. A DNA primer partially or totally as shown in Sequence No. 1, 2, 3 or 4 which specifically hybridizes to bovine male DNA fragments, obtainable from bovine male genomic DNA by using DNA probes.

6. A DNA sequence as shown in Sequence No. 5, 6 or 7 which hybridizes to both bovine male and female genomic DNA.

7. A DNA primer which comprises at least one part of DNA Sequence No. 5, 6 or 7 or its complementary strand and hybridizes to both bovine male and female genomic DNA.

8. A DNA primer as shown in DNA Sequence No. 8 or 9 which hybridizes specifically to bovine male DNA.

9. A DNA primer as shown in DNA Sequence No. 10 or 11 which hybridizes to both bovine male and female DNA.

10. A method of sexing bovine embryos by discriminating PCR products obtained by amplification of specific DNA sequences of the embryos, wherein a small part of an embryo provides DNA templates for the PCR, and two pairs of DNA primers are used for the PCR, one of which is chosen from DNA primers claimed in claim 4, 5 or 8 and the other of which is selected from those claimed in claims 7 or 9.

## Patentansprüche

1. DNA-Sequenz wie in Sequenz Nr. 1, 2 oder 3 dargestellt, die spezifisch mit männlicher genomischer Rinder-DNA hybridisiert.

2. DNA-Sequenz wie in Sequenz Nr. 4 dargestellt, die zwei Teile umfaßt, von denen einer spezifisch mit männlicher genomischer Rinder-DNA und der andere im allgemeinen mit männlicher und weiblicher DNA hybridisiert.

3. Sequenz wie teilweis oder vollständig in Sequenz Nr. 1, 2, 3 oder 4 dargestellt, erhältlich aus männlicher Rinder-DNA unter Verwendung von DNA-Sonden.

4. DNA-Primer, der mindestens einen Teil der DNA-Sequenz Nr. 1, 2, 3 oder 4 oder die komplementären Stränge umfaßt und spezifisch mit männlicher Rinder-DNA hybridisiert.

5. DNA-Primer, wie teilweise oder vollständig in Sequenz Nr. 1, 2, 3 oder 4 dargestellt, der spezifisch mit männlichen Rinder-DNA-Fragmenten hybridisiert, erhältlich aus männlicher genomischer Rinder-DNA unter Verwendung von DNA-Sonden.

6. DNA-Sequenz wie in Sequenz Nr. 5, 6 oder 7 dargestellt, die mit männlicher und weiblicher genomischer Rinder-DNA hybridisiert.

7. DNA-Primer der mindestens einen Teil der DNA-Sequenz Nr. 5, 6 oder 7 oder seinen komplementären Strang umfaßt und mit männlicher und weiblicher genomischer Rinder-DNA hybridisiert.

8. DNA-Primer wie in DNA-Sequenz Nr. 8 oder 9 dargestellt, der spezifisch mit männlicher Rinder-DNA hybridisiert.

9. DNA-Primer wie in DNA-Sequenz Nr. 10 oder 11 dargestellt, der mit männlicher und weiblicher Rinder-DNA hybridisiert.

10. Verfahren zur Geschlechtsbestimmung von Rinderembryos durch Unterscheiden der PCR-Produkte, die erhalten wurden durch Amplifikation spezifischer DNA-Sequenzen der Embryos, worin ein kleiner Teil eines Embryos DNA-Template für die PCR bereitstellt, und zwei Paare von DNA-Primern für die PCR verwendet werden, von denen eines ausgewählt ist aus in den Ansprüchen 4, 5 oder 8 beanspruchten DNA-Primern und das andere ausgewählt ist aus denen in den Ansprüchen 7 oder 9 beanspruchten.

## Revendications

1. Séquence d'ADN représentée par la Séquence n° 1, 2 ou 3, qui s'hybride spécifiquement à de l'ADN génomique de bovin mâle.

2. Séquence d'ADN représentée par la Séquence n°4, qui comporte deux parties dont l'une s'hybride spécifiquement à de l'ADN génomique de bovin mâle et l'autre s'hybride aussi bien à de l'ADN de bovin mâle qu'à de l'ADN de bovin femelle.

3. Séquence d'ADN représentée, en totalité ou en partie, par la Séquence n° 1, 2, 3 ou 4, que l'on peut obtenir à partir d'ADN de bovin mâle en utilisant des sondes d'ADN.

4. Amorce d'ADN qui comporte au moins une partie de l'une des Séquences d'ADN n° 1, 2, 3 et 4 ou de l'un des brins complémentaires, et qui s'hybride spécifiquement à de l'ADN de bovin mâle.

5. Amorce d'ADN représentée, en totalité ou en partie, par la Séquence n° 1, 2, 3 ou 4, qui s'hybride spécifiquement à des fragments d'ADN de bovin mâle et que l'on peut obtenir à partir d'ADN génomique de bovin mâle en utilisant des sondes d'ADN.

6. Séquence d'ADN représentée par la Séquence n° 5, 6 ou 7, qui s'hybride aussi bien à de l'ADN génomique de bovin mâle qu'à de l'ADN génomique de bovin femelle.

7. Amorce d'ADN qui comporte au moins une partie de l'une des Séquences d'ADN n° 5, 6 et 7 ou de l'un des brins complémentaires, et qui s'hybride aussi bien à de l'ADN génomique de bovin mâle qu'à de l'ADN génomique de bovin femelle.

8. Amorce d'ADN représentée par la Séquence d'ADN n° 8 ou 9, qui s'hybride spécifiquement à de l'ADN de bovin mâle.

9. Amorce d'ADN représentée par la Séquence d'ADN n° 10 ou 11, qui s'hybride aussi bien à de l'ADN de bovin mâle qu'à de l'ADN de bovin femelle.

10. Procédé de détermination du sexe d'embryons de bovins, par discrimination entre des produits de réaction en chaîne avec polymérase (PCR) obtenus par amplification de certaines séquences particulières de l'ADN des embryons, dans lequel procédé une petite partie d'un embryon fournit les modèles d'ADN utilisés dans la PCR, et l'on utilise dans la PCR deux paires d'amorces d'ADN dont l'une est choisie parmi les amorces d'ADN conformes à l'une des revendications 4, 5 et 8 et l'autre est choisie parmi les amorces d'ADN conformes à l'une des revendications 7 et 9.
